(19) Europäisches Patentamt
European Patent Office
Office européen des brevets

(11) EP 4 548 840 A1

(12) EUROPEAN PATENT APPLICATION

(43) Date of publication:
07.05.2025 Bulletin 2025/19

(51) International Patent Classification (IPC):
A61B 5/0205 (2006.01) A61B 5/11 (2006.01)
A61B 5/145 (2006.01) A61B 5/00 (2006.01)

(21) Application number: 24209691.5

(22) Date of filing: 30.10.2024

(52) Cooperative Patent Classification (CPC):
A61B 5/0205; A61B 5/1107; A61B 5/112;
A61B 5/1124; A61B 5/14546; A61B 5/4082;
A61B 5/4088; A61B 2560/0242

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.10.2023 GB 202316643
28.08.2024 DE 202024104839 U

(71) Applicant: Affotek Limited
Stockton-On-Tees, Durham TS21 3FB (GB)

(72) Inventor: Rajagopal, Chennakesavalu
Northwood, HA6 1RG (GB)

(74) Representative: Dekker, Lothar Karl Rudolf
Patent- und Rechtsanwaltskanzlei Dekker
Friedensheimer Straße 26
27729 Vollersode (DE)

(54) **WEARABLE SYSTEM**

(57) The present invention relates to a wearable system and a method for detecting and monitoring an indicator for a disease or a disease induced by an actual air pollution. The wearable system comprises at least one wearable device (1), to be worn on the body by a user. The at least one wearable device (1) comprising a plurality of integrated sensors (6) configured to detect biochemical and/or physiological vital signs of the user as well as environmental parameters indicative of an air pollution in the environment surrounding the user. The air pollutant may be selected from carbon monoxide, nitrous oxide, nitrogen dioxide, sulphur dioxide and particulate matter. In embodiments the air pollutant is carbon monoxide. In embodiments, the wearable device may be configured to detect user parameters such as fibrinogen levels in a user.

Fig. 5

EP 4 548 840 A1

## Description

Field of the Invention

The present disclosure relates generally to a wearable system for detecting and monitoring an indicator for a disease or a disease induced by an actual air pollution and wearables adapted thereto. The present invention relates to a wearable device for detecting pollution. In particular, the present invention relates to a wearable device for detecting pollutants such as carbon monoxide in the environment of a user and mediators that cause diseases as a result of exposure to indoor and outdoor pollutants. Furthermore, the invention relates to a method for detecting and monitoring an indicator for a disease or a disease induced by an actual air pollution.

Background to the Invention

Air quality is essential to the wellbeing of human beings. It is now accepted that air pollution contributes to ill health and death. Air pollutants are present both indoors and outdoors.

Household air pollution was estimated to be responsible for around 3.2 million deaths in 2020, including over 237,000 deaths of children under the age of 5 (WHO). Household air pollution is caused by smoking and the use of open fires and wood burning stoves, along with cooking indoors using open fires or inefficient stoves. Household air pollution can lead to the development of a number of diseases including stroke, heart disease, lung cancer and both chronic and acute respiratory diseases including asthma and COPD.

Air pollution outdoors is one of the greatest environmental risks to health and in 2019, it was estimated that 99% of the world's population was living in places where the WHO 25 air quality guidelines were not met (WHO). Outdoor air pollution is estimated to have caused 4.2 million premature deaths worldwide in 2019 and this is demonstrated by a coroner's court verdict in London on the death of a child with asthma (Ella Adoo-Kissi-Debrah: Air Pollution a factor in girl's death, Inquest finds: BBC News 16-12-2020).

Common pollutants include particulate matter, carbon monoxide, nitrogen dioxide and sulphur dioxide and are caused by incomplete combustion (carbon monoxide), and burning of fossil fuels and fuels for transport, for example. Similar to household air pollution, outdoor air pollution is a known cause of diseases such as ischaemic heart disease and stroke, COPD, cancers of the upper airways and lungs and respiratory diseases.

By reducing air pollution levels, countries could reduce the burden of diseases such as stroke, heart disease, lung cancer and both chronic and acute respiratory diseases including asthma (Ambient Air Pollution: Fact Sheet 19 Dec 2022 WHO).

Of the air pollutants, carbon monoxide is a colourless and tasteless gas that is released from exhausts of gasoline powered automobile vehicles, wood fires, stoves, forest fires, smoking tobacco in any form and bush fires. There are many other sources of carbon monoxide. Poor ventilation allows carbon monoxide levels to increase indoors.

Deaths as a result of high levels of carbon monoxide have been recorded in several countries, including around 116 deaths in 2020 in the UK (Office of National Statistics) and over 1300 deaths per year in North America in 2021 (Global Burden of Disease Study; Lancet, 2023).

Carbon monoxide was described by Dr Sally Davies, a former Chief Medical Officer of England & Wales as a 'silent killer' as its presence often goes undetected due to it being colourless, odourless and tasteless. Immediate effects of carbon monoxide poisoning include palpitations, nausea, shortness of breath, hallucinations and seizures. However, the symptoms are often subtle.

There are long-term effects of inhaling carbon monoxide such as memory loss, impaired cognition and permanent damage to the heart and brain. Carbon monoxide binds to haemoglobin, a protein that transports oxygen around the body. Carbon monoxide has a 200 times greater affinity for haemoglobin than oxygen and thus carbon monoxide displaces oxygen from haemoglobin to form carboxyhaemoglobin. By reducing the binding of oxygen to haemoglobin, the oxygen-carrying capability of the blood is decreased which can cause hypoxia and acidosis. Such lack of oxygen affects vital organs such as the brain and heart.

The effect of carbon monoxide can be detected by the amount of inhaled carbon monoxide and the reduced oxygen levels. Levels of carbon monoxide above 70 parts per million (ppm) cause dizziness, increased breathing rate, loss of consciousness, brain damage and death. However, levels of carbon monoxide above 5 ppm can affect the developing foetus. Of particular concern is the effect of carbon monoxide in pregnant mothers. Foetal haemoglobin has a particular affinity for carbon monoxide, which results in the foetus being deprived of oxygen more severely than the mother. Premature birth, death of the foetus and damage to the infant's brain are reported following exposure to carbon monoxide. Carbon monoxide levels are monitored at antenatal appointments using a breath monitor, which indicates the ppm of carbon monoxide or percentage of carboxyhaemoglobin present in the breath. However, these monitors are only used intermittently at antenatal appointments and therefore do not immediately alert the mother to the presence of carbon monoxide and/or carbon monoxide poisoning. In order to be useful to pregnant women, due to the high affinity of foetal haemoglobin for carbon monoxide, very low levels of carbon monoxide need to be detected using any monitor.

Measurement of carboxyhaemoglobin is an accurate way of assessing the severity of carbon monoxide poisoning and can be measured using pulse oximetry or, more accurately with a CO-oximeter, which measures the carbon monoxide bound to haemoglobin in a blood sample. CO-oximeters measure the absorption of light passing through the blood in order to distinguish between oxyhaemoglobin, deoxyhaemoglobin and carboxyhaemoglobin.

It is an object of the present invention to obviate or mitigate one or more of the abovementioned problems and/or provide a device, which allows convenient and/or rapid identification of environmental pollutants, particularly carbon monoxide.

Summary of the Invention

The present invention relates to a wearable system comprising at least one wearable device for monitoring an indicator for a disease or a disease induced by an actual air pollution and is based, in part, on studies by the inventor in which he has shown it is possible to accurately measure, among other things, the level of the pollutant carbon monoxide at low levels using a wearable device. Furthermore, the invention relates to a method for detecting and monitoring an indicator for a disease or a disease induced by an actual air pollution. The wearable system comprising at least one wearable device with at least one sensor, which is enabled to detect pollutants and mediators that cause diseases as a result of exposure to indoor and outdoor pollutants. Other features of the invention will be apparent from the dependent claims, and the description which follows.

In a first aspect of the present invention there is provided a wearable system for detecting and monitoring an indicator for a disease or a disease induced by an actual air pollution, the system comprising:

at least one wearable device, to be worn on the body by a user;

the at least one wearable device comprising a plurality of sensors, attached to the wearable device, configured to detect biochemical and/or physiological vital signs of the user as well as environmental parameters indicative of an air pollution in the environment surrounding the user;

each wearable device is configured to transmit sensor data to a computerised device;

the computerised device comprising at least one processor, a non-transitory memory, and at least one user interface;

the computerised device analyses and monitors said vital signs and environmental parameters detected by the wearable device;

the computerised device is configured to generate an alert if at least one threshold for the detected biochemical and/or physiological vital signs and/or the detected environmental parameter is exceeded.

The wearable system for detecting and monitoring an indicator for a disease or a disease induced by an actual air pollution enables the detection of vital signs, environmental pollution such as particulate matter or gases such as carbon monoxide and diseases caused by environmental pollutants. The at least one wearable device includes sensors enabled to detect pollutants such as carbon monoxide, carbon dioxide, particulate matter, both small and large, sulphur dioxide, nitrous oxide, nitrogen dioxide, circulating fibringen and interleukin. All the sensors communicate to the computerised device worn by the user securely by means of secure Bluetooth or Wi-Fi. Such wearable device is of use to detect a variety of diseases that include heart diseases, Parkinson's disease and cancer induced by long-term exposure to pollution, protect the vulnerable, detect fatigue, drowsiness and improve the lives of those exposed to pollution and living alone.

Preferably, the wearable device worn by the user is configured to monitor the gait of the user. A gait analysis can performed by means of measuring the horizontal distance of movement of legs, the speed, posture, pressure exerted vertically, laterally and direction of the leg perpendicular to the floor with each step of the user. Such a methodology allows analysis of the user's gait. A suitable combination of wearable devices allows distinguishing various types of gait. By way of example, combining information received by a ring and an anklet as wearable devices it may be possible to distinguish the gait in Parkinson's disease from Dementia and Alzheimer's.

In another embodiment, that combines the ring and the ear loop as wearable devices, detection of fatigue due to chronic inhalation of indoor and outdoor pollutant is enabled using such sensors that can detect activity, muscle weakness as characterised by changes in amplitude and frequency of muscle fibre contraction.

In embodiments, the computerised device may comprise a computerised algorithm configured to analyse the activity of the user by measurement of muscle contractility, wherein the detection of amplitude and frequency of such muscle movements results in decision making based on artificial intelligence, machine learning and deep learning. The variation in amplitude and frequency of muscle movements helps to characterise muscle diseases as in fatigue detection, Parkinson's disease and dementia. By way of example, in a medical illness called Parkinson's disease, the muscle contractions are of higher amplitude and lower frequency, whereas the muscle contraction in another medical condition known as essential tremor, the muscle contraction is of lower amplitude and the frequency is higher. A low frequency and high amplitude is to be found in multiple sclerosis.

In embodiments, the computerised device may comprise a computerised algorithm configured to analyse muscle movement, amplitude and frequency of muscle contraction and gait to arrive distinguishing various types of gait based on artificial intelligence, machine learning and deep learning.

In embodiments, the computerised device comprises a computerised algorithm configured to analyse movements, which may a tremor of fingers, wherein the detection of amplitude and frequency of such finger movements results in decision-making based on artificial intelligence, machine learning and deep learning.

In embodiments, the computerised device is adapted to detect carbon monoxide by means of a spectrophotometric method.

In embodiments, the computerised device is adapted to determine the presence of carboxyhaemoglobin value based on a wavelength measurement of oxyhaemoglobin and methaemoglobin.

In embodiments of the system at least one of the sensors being is configured to detect a biomarker. Mediators of diseases caused by environmental pollution are also known as biomarkers. These may be carboxy haemoglobin in carbon monoxide inhalation. Furthermore, proteins and messenger substances, in particular fibrinogen and interleukin, especially interleukin-6, acting as mediators of damage caused by pollution, both indoors and outdoors.

The wearable device for detecting and/or monitoring an indicator for a disease or a disease induced by an actual air pollution in the environment of a user comprises a plurality of sensors attached to the wearable device, whereby the sensors being configured to detect an air pollutant in the environment of the user.

In embodiments, the sensors are both miniaturised mechanical and electrochemical elements.

The wearable system of the present invention is, among other, able to detect the presence of an air pollutant in the environment of a user of the wearable device. The term "air pollutant" as used herein is used to mean airborne pollutants present in the environment which cause harm to humans. The pollutants may be particulates of various sizes, gases or vapours, for example. The air pollutant may be selected from: carbon monoxide, carbon dioxide, nitrous oxide, nitrogen dioxide, sulphur dioxide and particulate matter. Pollutants also include lead and ozone, for example. In embodiments, the air pollutant is carbon monoxide.

The present inventor has shown that the wearable system of the present invention is able to accurately and quickly inform a user of the device that carbon monoxide is present in the environment, even at low levels. Advantageously, a user can therefore quickly address the presence of carbon monoxide and/or remove themselves from the area to avoid carbon monoxide poisoning. Such early detection is advantageous as the presence of carbon monoxide can be identified before any adverse effects become apparent. Such a wearable system may be particularly useful to pregnant women, since the developing foetus is particularly at risk of very low levels of carbon monoxide, as well as people working in industries where exposure to carbon monoxide is possible, for example gas engineers. What is more, the wearable system may be used by the general public to provide information regarding the environmental pollutants and enable then to adjust their exposure to pollutants accordingly.

The term "environment of the user" as used herein is used to mean the surroundings of the user at the time the user is wearing the wearable device of the wearable system. The environment may be indoors or outdoors. For example, the environment may be the home or workplace of the user. The environment may be outdoors while travelling, taking exercise or commuting, for example.

The term "detecting" as used herein is used to mean identify the presence of the air pollutant. The wearable device may be able to detect the exact level of the air pollutant in the user's environment.

The term "monitoring" as used herein is used to mean observe the presence or absence of the air pollutant. The monitoring may be done over time such that the level of the air pollutant over time can be analysed.

As will be understood by the skilled person a wearable device is a device which can be worn as an accessory of the user or embedded in the clothes of the user, for example. The device may be any suitable wearable device. However, in embodiments, the wearable device comprises a ring, armlet, armband, anklet, legband, wrist strap, headband or ear loop. For example, the wearable device configured as an anklet can be clasped around the ankle or lower leg. The device configured as an ear loop sits around the lobule of ear. Such wearable devices are easy to attach to the user and comfortable to wear. By using a wearable device such as the ones listed, user compliance is increased due to ease of use. The skilled person will be aware of wearable devices. The term ear loop as used herein means a loop which sits around the lobule of the ear.

The wearable device may be composed of any suitable material. Preferably, the material is a sustainable or recyclable material such as textile and metal (rather than plastic). In embodiments, the wearable device may be composed of a metal, for example stainless steel or titanium. Alternatively, the wearable device may be composed of a textile, for example electronic textile. Electronic textiles include, for example, e textile where the sensor is fabricated into the textile. In embodiments where the device is a ring, a metal may be titanium or silver. Alternatively, in embodiments in which the device is an armlet, armband, anklet, legband, wrist strap, headband a more flexible textile material may provide advantages as the sensor could be fabricated and the device washable.

The wearable device may comprise a frame to which the sensors are attached. In embodiments, the sensors may be moulded into the frame or integrated into the frame. By "integrated" or "moulded" into the frame, it is to be understood that the sensor is held within the frame material and not simply attached to a surface of the frame. This may allow the sensor to be more securely integrated and less likely to be detached from the frame.

According to the invention, the wearable device of the wearable system comprise a plurality of sensors. The multiple sensors may detect a single pollutant type, for example carbon monoxide. Alternatively, one sensor may detect a single pollutant type, for example carbon monoxide and/or a second sensor may detect a second pollutant type, for example particulate matter and/or a third sensor may detect a third pollutant type, for example nitrous oxide. In embodiments, the sensors may detect diverse biochemical substances such as carboxyhaemoglobin or fibrinogen or interleukin, a protein and physiological vital signs. The sensors are both miniaturised mechanical and electrochemical elements.

At least one of the sensors may configured to detect a user parameter, optionally wherein the user parameter is selected from: fibrinogen level, interleukin level, blood pressure, heart rate, breathing rate, respiratory rate, skin temperature and blood oxygen level. The plurality of sensors in wearable device enables to detect carbon monoxide, carbon dioxide, particulate matter, sulphur dioxide, nitrous oxide and fibrinogen levels and/or interleukin levels in circulating blood based on the optical wavelength of these, among other things. Additionally, such wearable device is enabled to detect health impacts of such pollutants.

The sensors of the present invention may comprise an LED of photonic origin or may be formed from graphene, graphene oxide or a silicon composition. Preferably, the sensors uses plain graphene or graphene oxide or photonic diode as source of light for measurement of pollutants and substances that pollutants induce in the body called biomarkers. For example, the wearable device configured as ear loop may include an electrode made of plain graphene or graphene oxide as a sensor to detect brain waves.

The air pollutants may be detected directly or indirectly. In embodiments in which the pollutant is carbon monoxide the sensor may detect carboxyhaemoglobin in the user. This is indirect detection of the carbon monoxide. Measurement of carboxy haemoglobin, which is the amount of oxygen that is bound carbon monoxide, is an accurate way of assessing the severity of carbon monoxide poisoning. The carbon monoxide sensor may be a sensor detecting the concentration of carbon monoxide by optical absorbance. Thus in such embodiments, the sensor may be an optical sensor. Such detection would be used based on the absorption of light passing through the user in order to distinguish between oxyhaemoglobin, deoxyhaemoglobin and carboxyhaemoglobin. Absorption of light may be detected at one or more of the following wavelengths: 420 nm (oxyhaemoglobin), 432 nm (methaemoglobin), 540 nm, 579 nm.

Such measurements can be used to determine the level of carboxyhaemoglobin (COHb%) using the following equation:

$$COHb\% = 100*(C-B)/(A-B)$$

where:

B = 0% COHb peak ratio at a wavelength of 540 nm and 579 nm;

A = 100% COHb peak ratio at a wavelength of 540 nm and 579 nm; and

C = the peak ratio at a wavelength of 540 nm and 579 nm in the blood of the user.

Such sensors would enable extremely accurate and direct contemporary readings and at low levels of carbon monoxide. The use of such sensors in the present invention would improve usage in both on the spot measurement and continuous monitoring and quickly alert the user as a way of preventing health issues developing.

It will be appreciated that in alternative embodiments or in addition, the carbon monoxide sensor may detect carbon monoxide in the air. Such detection would be direct detection of the carbon monoxide.

In embodiments in which the pollutant comprises carbon dioxide, the sensor may comprise an infrared sensor. In embodiments in which the pollutant comprises nitrous oxide the sensor may detect light absorption (enabling detection of 25 ppm).

In embodiments in which the pollutant comprises particulate matter the sensor may detect intensity of light scattering in the region of 90° and/or 45° and/or at an angle or angles therebetween. In embodiments in which the pollutant comprises nitrogen dioxide the sensor may comprise an electrochemical sensor. In embodiments in which the pollutant comprises sulphur dioxide the sensor may comprise an electrochemical sensor.

In embodiments of the present invention, at least one sensor of the wearable device is configured to detect a user parameter.

The term "user parameter" as used herein means a physiological characteristic of the user of the wearable device. As discussed above, air pollution contributes to ill health and death. Therefore, it is advantageous if the wearable device can detect physiological characteristics of the user which are indicative of health and/or disease. For example, the user parameter may be selected from blood pressure, heart rate, breathing rate, respiratory rate, skin temperature and blood oxygen level. A person with asthma or illness affecting blood vessels or heart (as in high blood pressure) may be susceptible to lower levels of pollutants and therefore observing user parameters in addition to pollutant levels may be beneficial. Other user parameters may include activity of the user, for example muscle activity. By sensing user parameters which are indicative of health and/or disease it is possible for the wearable device of the present invention to be used to determine adverse health effects associated with exposure to pollutants. The device may be used to alert the user if a user parameter exceeds a particular threshold and the user could then take action accordingly.

In embodiments, at least one sensor in the wearable device may be configured to detect fibrinogen and/or interleukin in the user. Fibrinogen is a glycoprotein used in the risk assessment and diagnosis of diseases of cardiac, metabolic, immune and cancer origin. Fibrinogen is also a marker of diseases affecting the heart, cancer and a group of autoimmune diseases. Interleukin is a peptide hormone belonging to the cytokines, i.e. they are the body's own messenger substances of the cells of the immune system. Interleukins circulate in the blood stream of the user. In particular interleukin-6 (IL-6) which regulate the inflammatory reaction of the organism, causes the increased synthesis of acute phase proteins in the liver. It is important in the prognosis and assessment of trauma and sepsis.

Pollution induces all such diseases and fibrinogen can therefore be used to detect damage caused by outdoor pollutants such as coronary artery disease or cancer in its early stages.

Fibrinogen levels are usually detected using enzyme linked immunosorbent assay (ELISA), radio immunoassay (RIA) or electro chemiluminescence immunoassay (ECLIA). However, such detection requires sophisticated laboratory equipment and does not give an immediate indication of fibrinogen levels.

In the present invention, when fibrinogen levels and/or interleukin levels, especially interleukin-6 levels, of the user are detected, at least one of the sensors may be an optical sensor. The sensor may detect absorbance at 540 nm to detect and quantify the fibrinogen levels. The sensor may detect absorbance in the region of 254 nm to detect and quantify the interleukin levels. The sensor may be a plain graphene or graphene oxide sensor or another light source or silicone composition.

In embodiments, the wearable device detects both indoor and outdoor pollutants. By detecting both carbon monoxide and fibrinogen and/or interleukin levels, the wearable device of the invention is advantageously able to detect pollutants in both indoor and outdoor environments.

The wearable device may further comprise a computerised device. The computerised device may be configured to communicate with the sensors to receive data from the sensors. The data may relate to the pollutant or user parameters detected or monitored by the sensors.

The computerised device may comprise one or more of a processor, non-transitory memory and a user interface/display.

The processor may be arranged to process air pollutant and/or user parameter data received from the sensors; and analyse the air pollutant and/or user parameter data by comparing an air pollutant and/or user parameter value with a threshold value.

The processor may comprise one or more functional or physical parts, for example one for processing the data, one for analysing the data, etc. In embodiments, the processor may be further arranged to provide an output indicating whether an air pollutant is present and/or providing information regarding a user parameter. In embodiments, the output may be provided on a display/user interface.

The processor and display/user interface may be integrated in a single device. The wearable device may also be integrated into said single device. However, in alternative embodiments, the wearable device, processor and display may each be single devices which are interconnected.

The computerised device may be integral with the wearable device. Alternatively, the computerised device may comprise a second device. In embodiments, the computerised device may be a phone or tablet of the user, for example. The computerised device may be a wearable device.

The computerised device may comprise a user interface. As will be appreciated by the skilled person a user interface, or display, allows the user to obtain information from the computerised device. The information displayed on the interface may relate to the pollutant or user parameter readings obtained by the sensors, for example.

As set out above, the computerised device may be configured to analyse the data obtained from the sensors. The computerised device may generate an alert (which may be displayed on the user interface) if a threshold value for the pollutant or user parameter is exceeded. The alert may be a visual or audible alert. For example, where the air pollutant is carbon monoxide, the computerised device may generate an alert when a level of carbon monoxide over 2 ppm, 3 ppm, 4 ppm, 5 ppm, 10 ppm, 15 ppm, 20 ppm, 25 ppm, 30 ppm, 35 ppm, 40 ppm, 45 ppm or 50 ppm is reached. The threshold value may be altered depending on the user. For example, for pregnant women, a low threshold value of 5 ppm may be advantageous due to the high binding affinity of carbon monoxide for foetal haemoglobin. Suitable threshold values may be set depending on the pollutants and/or user parameters which are to be detected.

By providing an alert to the user, the user is quickly informed of the presence of a pollutant or harmful user parameter and can act accordingly, for example by moving to a different environment or seeking the input of a health professional.

The wearable device and/or computerised device may comprise wireless communication modules which allow the sensor to communicate with the computerised device to provide data to said computerised device.

The present invention also provides a method for detecting and monitoring an indicator for a disease or a disease induced by an actual air pollution, the method comprising:

- using at least one wearable device, worn on the body by a person;

- using a plurality of sensors attached to said wearable device, to detect biochemical and/or physiological vital signs of the person as well as environmental parameters indicative of an air pollution in the environment surrounding the person, and generating, by the plurality of sensors, sensor data corresponding to said biochemical and/or physiological vital signs and/or said environmental parameters;

- transmitting, by said wearable device, the sensor data to a wearable computerised device, the wearable computerised device comprising at least one processor, a non-transitory memory, and at least one user interface;

- receiving, by the wearable computerised device, the sensor data so that the wearable computerised device generates an alert if at least one threshold for the detected biochemical or physiological vital signs or the detected environmental parameter is exceeded.

Also disclosed is the use of a device according to the present invention for detecting and/or monitoring air pollution in the environment of a user.

Detailed Description of the Invention

The present invention will now be further described with reference to the following figures which show:

Figure 1: A wearable ring device for detecting carbon monoxide;
Figure 2: An alternate wearable pollution detection device;
Figure 3: An alternate wearable pollution detection device;
Figure 4: An alternate wearable pollution detection device; and
Figure 5: is a block diagram of an embodiment of the system.

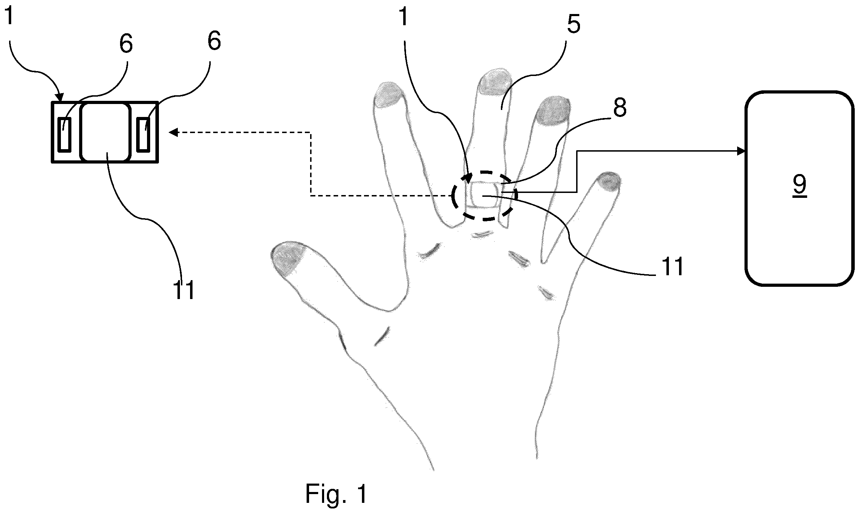

The present inventor has undertaken significant experimentation to develop the present invention. Figure 1 shows a wearable device 1 as part of a wearable system according to the invention. The wearable device 1 in this example is in the form of a ring to be worn on a user's finger 5. The ring is formed of a sustainable and recyclable metal. The ring forms a frame into which a plurality of sensors 6 are moulded within the shank 8 (the metal frame covering the phalanx of the finger) on either side of the finger 5. In this example, the sensors 6 are able to detect ambient carbon monoxide levels/carboxyhaemoglobin levels. The plurality of sensors 6 are enabled to detect the level of carbon monoxide that is bound to haemoglobin, a protein molecule in the circulating blood that carries oxygen. This is termed carboxy haemoglobin. Such a method of continuous detection method enables early identification of those exposed to excessive levels of carbon monoxide. In this example, the sensor 6 is made from graphene. In addition, sensors may be present which are able to detect activity, blood pressure, heart rate, respiratory rate, temperature and blood oxygen levels of the user.

The sensors 6 communicates the data obtained with a wearable computerised device 9. The computerised device 9 may be a mobile phone or watch, for example. The wearable device 1 of the invention may be connected to the computerised device 9 by way of secure Bluetooth or Wi-Fi. The computerised device 9 analyses and monitors the data 5 received from the wearable device 1 and may generate an alert if a threshold value for the detected pollutant or other parameter of interest is exceeded. The alert will notify the user of the threshold value being exceeded such that they can take mitigating action, for example moving to an alternative location to avoid the pollutant.

On the dorsal aspect of the hand commonly known as the back of the hand, is the top part of the ring, called the head. The head here is fabricated from transparent touch sensitive material that displays values on user interface 11. Such values may be chosen by the individual wearer to display any particular function as required or desired by such individual. It may also be possible to change the particular function. By way of example, tapping the surface on the head of the ring, it may be possible to show detection of the carbon monoxide or other pollutant or user parameters such as the heart rate, breathing rate, respiratory rate, blood pressure, oxygen levels in the blood and the skin temperature of wearer.

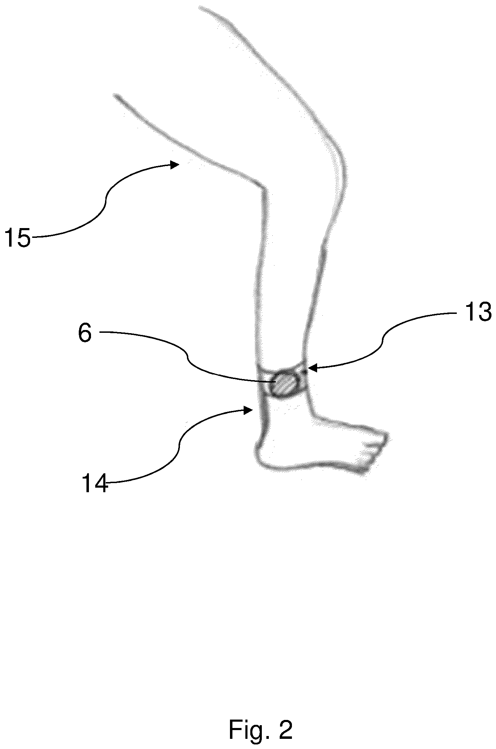

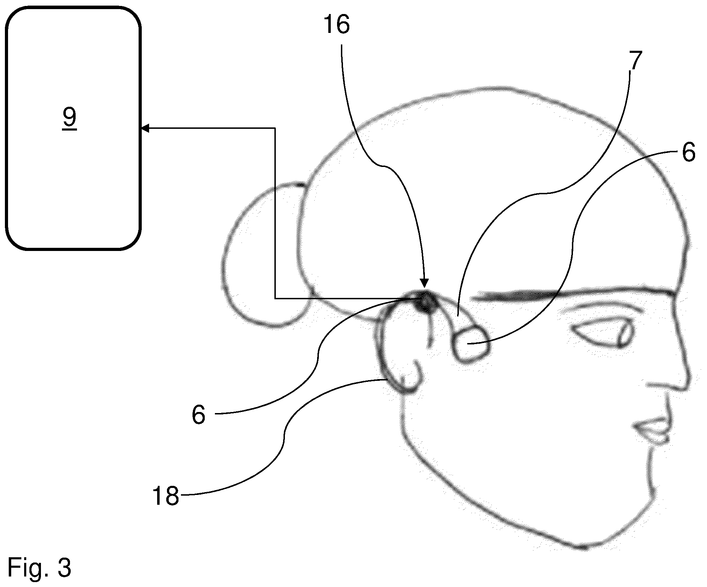

In an alternate device, as shown in Figure 2, the device may be in the form of an anklet or leg strap 13. The wearable device 1 is formed of sustainable and recyclable metallic or textile material which encircles the ankle 14 or the leg 15. A sensor 6 in the anklet 13 detects the presence of carbon monoxide. In addition, additional sensors 6 (not shown) in the anklet detect heart rate, respiratory rate and blood pressure. Similar to the device shown in Figure 1, the device of Figure 2 can detect the pollutant carbon monoxide and communicate the data obtained with a computerised device 9 (as shown in Fig. 3). The computerised device 9 analyses the data and may generate an alert for the user if a threshold value for the carbon monoxide is exceeded. The anklet 13 that encircles the ankle allows detection of effects on infants and children exposed to low levels of pollution. These may be carbon monoxide or particulate matter also called aerosol. It is made possible by sensors 6 that detect carboxyhaemoglobin and fibrinogen, a protein.

Figure 3 shows a further alternate device in which the device comprises an ear loop 16. The ear loop 16 sits above the lobule of the ear 18 with an extension 7 that abuts the facial skin above the zygoma. The device 1 includes a sensor 6 integrated into the ear loop 16 which detect one or more pollutants in the environment, in this case carbon monoxide. In this example, one of the sensors 6 also detects a user parameter, in this case the fibrinogen level. The sensor 6 communicates the data obtained with the computerised device 9. The computerised device 9 may be a mobile phone or watch, for example. The wearable device 1 may be connected to the computerised device 9 by way of secure Bluetooth or Wi-Fi. The computerised device 9 analyses and monitors the data 5 received from the wearable device 1 and may generate an alert if a threshold value for the detected pollutant or other parameter of interest is exceeded. The alert will notify the user of the threshold value being exceeded such that they can take mitigating action, for example moving to an alternative location to avoid the pollutant. A combination of the ring and the ear loop allows the detection of fatigue due to chronic inhalation of indoor and outdoor pollutant. This is made possible by means of sensors that detect activity, muscle weakness as characterised by changes in amplitude and frequency of muscle fibre contraction. According to a further embodiment, the upper part of the ear loop 16 that is above the lobule of the ear 18 is fitted with graphene electrodes 12 to detect brain waves emerging from the temporal area of the brain. Such an electrode 12 is enabled with Bluetooth, amplification and filtering process to remove artefacts caused by movements and noise. It establishes communication with the computerised device 9. The lower end of the ear loop 16 curves to hold a sensor 6 that is pressing against the skin of the face above the zygoma or malar bone. The sensors 6 detects the blood pressure, oxygenation, heart rate and respiratory rate. The internal maxillary artery is lodged in zygomatic fossa which is a cavity just above and behind the zygoma or malar bone. This would allow the wearable device 1 to detect circulating fibrinogen and interleukin 6.

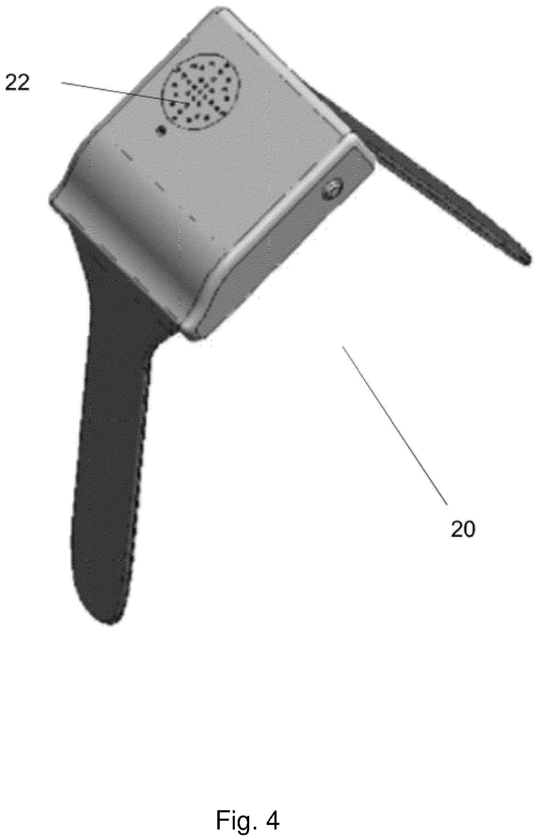

Figure 4 shows a further alternative device. In this example, the wearable device 20 takes the form of a wrist strap containing an integrated sensor (not shown), computerised device (not shown) and alert system 22. The sensor detects the presence of a pollutant, for example carbon monoxide and communicates with the computerised device 9 which analyses the data and provides an alert to the user if a threshold value for the pollutant is exceeded.

The inventor has undertaken testing of the device of Figure 4 and shown that the device can detect pollutants, such as carbon monoxide at low levels. Such a device is particularly useful for pregnant women, for example, where detection of low levels of carbon monoxide is essential due to the high affinity of carbon monoxide for foetal haemoglobin.

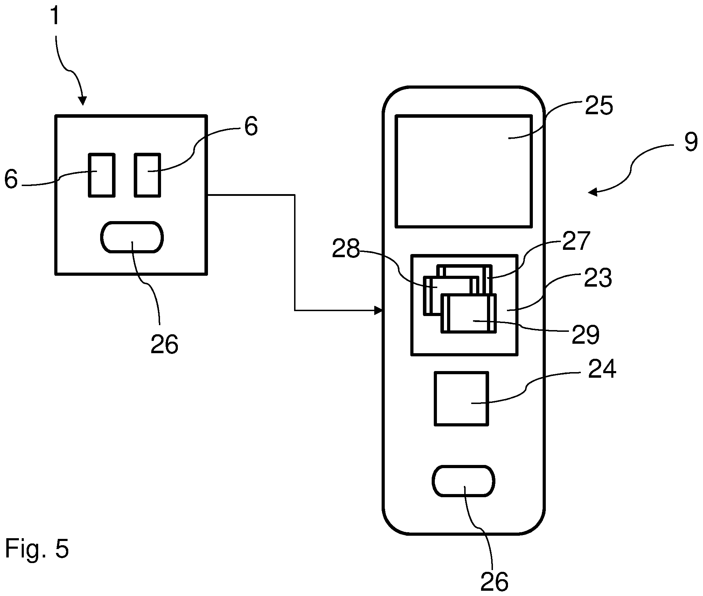

Figure 5 shows an embodiment of the wearable system for detecting and monitoring an indicator for a disease or a disease induced by an actual air pollution.

The wearable system comprises at least one wearable device 1 as described in detail above, to be worn on the body by a user. The at least one wearable device 1 comprises a plurality of integrated sensors 6 as described above. The integrated sensors 6 are configured to detect biochemical and/or physiological vital signs of the user as well as environmental parameters indicative of an air pollution in the environment surrounding the user.

Each wearable device 1 is configured to transmit sensor data to the computerised device 9. The computerised device 9 comprises at least one processor 23, a non-transitory memory 24, and at least one user interface 25. The computerised device 9 analyses and monitors said vital signs and environmental parameters detected by the wearable device 1.

The computerised device 9 is configured to generate an alert if at least one threshold for the detected biochemical and/or physiological vital signs and/or the detected environmental parameter is exceeded.

The wearable device 1 of the invention may be connected to the computerised device 9 by way of secure Bluetooth or Wi-Fi. The wearable device 1 and the computerised device 9 both have a communication interface 26 for this purpose.

The computerised device 9 comprises a computerised algorithm 27, which is configured to analyse the activity of the user by measurement of muscle contractility, wherein the detection of amplitude and frequency of such muscle movements results in decision making based on artificial intelligence, machine learning and deep learning.

In a further aspect, the computerised device 9 comprises a computerised algorithm 28, which is configured to analyse muscle movement, amplitude and frequency of muscle contraction and gait to arrive distinguishing various types of gait based on artificial intelligence, machine learning and deep learning.

According to another aspect, the computerised device 9 comprises a computerised algorithm 29, which is configured to analyse movements, in particular a tremor of fingers, wherein the detection of amplitude and frequency of such finger movements results in decision-making based on artificial intelligence, machine learning and deep learning.

It will be appreciated that numerous modifications to the above described wearable system, device and method may be made without departing from the scope of the invention as defined in the appended claims. For example, although the example devices shown comprise a ring, anklet, ear loop and wrist strap/bracelet it will be appreciated that other wearable devices are within the scope of the invention, for example an armlet or headband.

Throughout this specification, the term "comprising" or "comprises" means including the component(s) specified but not to the exclusion of the presence of other components. The term "consisting essentially of" or "consists essentially of" means including the components specified but excluding other components except for materials present as impurities, unavoidable materials present as a result of processes used to provide the components, and components added for a purpose other than achieving the technical effect of the invention.

The term "consisting of" or "consists of" means including the components specified but excluding addition of other components.

Whenever appropriate, depending upon the context, the use of the term "comprises" or "comprising" may also be taken to encompass or include the meaning "consists essentially of" or "consisting essentially of", and may also be taken to include the meaning "consists of" or "consisting of".

The optional features set out herein may be used either individually or in combination with each other where appropriate and particularly in the combinations as set out in the accompanying claims. The optional features for each aspect or exemplary embodiment of the invention as set out herein are also to be read as applicable to any other aspect or exemplary embodiments of the invention, where appropriate. In other words, the skilled person reading this specification should consider the optional features for each exemplary embodiment of the invention as interchangeable and combinable between different exemplary embodiments.

All of the features disclosed in this specification (including any accompanying claims, and drawings), and/or all of the steps of any method so disclosed, may be combined in any combination, except combinations where at least some of such features and/or steps are mutually exclusive.

Each feature disclosed in this specification (including any accompanying claims, and drawings) may be replaced by alternative features serving the same, equivalent or similar purpose, unless expressly stated otherwise. Thus, unless expressly stated otherwise, each feature disclosed is one example only of a generic series of equivalent or similar features.

References

Ella Adoo-Kissi-Debrah: Air Pollution a factor in girl's death, Inquest finds: BBC News 16-12-2020

Ambient Air Pollution: Fact Sheet 19 Dec 2022 World Health Organisation Global, regional, and national mortality due to unintentional carbon monoxide poisoning, 2000-2021: results from the Global Burden of Disease Study 2021, Lancet 10 Public Health 2023; 8: e839-49

**Claims**

1. A wearable system for detecting and monitoring an indicator for a disease or a disease induced by an actual air pollution, the system comprising:

   - at least one wearable device (1), to be worn on the body by a user;
   - the at least one wearable device (1) comprising a plurality of integrated sensors (6) configured to detect biochemical and/or physiological vital signs of the user as well as environmental parameters indicative of an air pollution in the environment surrounding the user;
   - each wearable device (1) is configured to transmit sensor data to a computerised device (9);
   - the computerised device (9) comprising at least one processor (23), a non-transitory memory (24), and at least one user interface (25);
   - the computerised device (9) analyses and monitors said vital signs and environmental parameters detected by the wearable device (1);
   - the computerised device (9) is configured to generate an alert if at least one threshold for the detected biochemical and/or physiological vital signs and/or the detected environmental parameter is exceeded.

2. The wearable system according to claim 1, wherein the air pollutant is selected from: carbon monoxide, carbon dioxide, nitrous oxide, nitrogen dioxide, sulphur dioxide and particulate matter.

3. The wearable system according to claim 1 or 2, wherein the sensors (6) comprises an LED of photonic origin or is formed from graphene, graphene oxide or silicon.

4. The wearable system according to any preceding claim wherein at least one of the sensors (6) is configured to detect a user parameter, optionally wherein the user parameter is selected from: fibrinogen level based on wavelength of 540nm, interleukin level based on wavelength of 254 nm, blood pressure, heart rate, breathing rate, respiratory rate, skin temperature and blood oxygen level.

5. The wearable system according to any preceding claim wherein the wearable device (1) further comprises a computerised device (9) configured to communicate with the sensors (6) to receive data from the sensors (6).

6. The wearable system according to any preceding claim wherein the sensors (6) are both miniaturised mechanical and electrochemical elements.

7. The wearable system according to claim 5 or 6 wherein the computerised device (9) is configured to analyse said data and generate an alert if a threshold value for the pollutant or user parameter, in particular biomarker, is exceeded.

8. The wearable system according to any preceding claim, wherein the computerised device (9) comprises a computerised algorithm configured to analyse the activity of the user by measurement of muscle contractility, wherein the detection of amplitude and frequency of such muscle movements results in decision making based on artificial intelligence, machine learning and deep learning.

9. The wearable system according to any of the preceding claims, wherein the computerised device (9) comprises a computerised algorithm configured to analyse muscle movement, amplitude and frequency of muscle contraction and gait to arrive distinguishing various types of gait based on artificial intelligence, machine learning and deep learning.

10. The wearable system according to any of the preceding claims, wherein the computerised device (9) comprises a computerised algorithm configured to analyse movements, in particular a tremor of fingers, wherein the detection of amplitude and frequency of such finger movements results in decision-making based on artificial intelligence, machine learning and deep learning.

11. The wearable system according to any of the preceding claims, wherein the computerised device (9) is adapted to detect carbon monoxide by means of a spectrophotometric method.

12. The wearable system according to claim 11, wherein the computerised device (9) is adapted to determine the presence of carboxyhaemoglobin value based on a wavelength measurement of oxyhaemoglobin and methaemoglobin.

13. The wearable system according to any of the preceding claims, wherein the level of carboxyhaemoglobin is calculated according the equation:

$$COHb\% = 100*(C-B)/(A-B)$$

where B=0% COHb denotes a value for peak ratio at 540 nm and 575 nm; A=100% COHb denotes a value for peak ratio at 540 nm and 579 nm; and C denotes a value for peak ratio at 540 nm and 579 nm in the blood of the user.

14. The wearable system according to any of the preceding claims, wherein the computerised device (9) is adapted to analyse detected biomarker level, measurements of activity and measurements of brain activity by electroencephalogram by means of the wearable device (1), in particular the ear loop as the wearable device (1), wherein the evaluation of a combination of measured biomarker values, measured activity and EEG measurement of the person by the computerised device (9) of the wearable system enables the detection of dementia and Parkinson's disease.

15. A method for detecting and monitoring an indicator for a disease or a disease induced by an actual air pollution, the method comprising:

   - using at least one wearable device (1), worn on the body by a person;
   - using a plurality of sensors (6) attached to said wearable device, to detect biochemical and/or physiological vital signs of the person as well as environmental parameters indicative of an air pollution in the environment surrounding the person, and generating, by the plurality of sensors (6), sensor data corresponding to said biochemical and/or physiological vital signs and/or said environmental parameters;
   - transmitting, by said wearable device (1), the sensor data to a wearable computerised device (9), the wearable computerised device (9) comprising at least one processor, a non-transitory memory, and at least one user interface;
   - receiving, by the wearable computerised device (9), the sensor data so that the wearable computerised device (9) generates an alert if at least one threshold for the detected biochemical or physiological vital signs or the detected environmental parameter is exceeded.

Fig. 1

Fig. 2

Fig. 3

22

20

Fig. 4

Fig. 5

EP 4 548 840 A1

Europäisches
Patentamt

European
Patent Office

Office européen
des brevets

# EUROPEAN SEARCH REPORT

**Application Number**

EP 24 20 9691

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/174778 A1 (GOLDSTEIN STEVEN WAYNE [US]) 10 June 2021 (2021-06-10) <br> * paragraphs [0065] - [0066], [0068], [0084] - [0088], [0101], [0132] - [0133], [0155], [0173], [0218], [0220], [0222], [0231] * <br> * figures 1A-1B * | 1-15 | INV. <br> A61B5/0205 <br> A61B5/11 <br> A61B5/145 <br> A61B5/00 |
| X | US 2022/084672 A1 (HALL GERARD J [US]) 17 March 2022 (2022-03-17) <br> * paragraphs [0043] - [0046], [0061], [0074] - [0075] * <br> * figures 1-2 * | 1-15 | |
| X | US 2017/351221 A1 (BALTI HAIKEL [FR] ET AL) 7 December 2017 (2017-12-07) <br> * paragraphs [0009] - [0010], [0014], [0021], [0034] - [0036], [0069] - [0072], [0136], [0140], [0143] - [0147], [0155] * <br> * figures 1-7 * | 1-15 | |
| X | CN 109 770 863 A (UNIV TIANJIN) 21 May 2019 (2019-05-21) <br> * paragraphs [0005] - [0019], [0024] - [0036] * <br> * figure 1 * | 1-15 | TECHNICAL FIELDS SEARCHED (IPC) <br><br> A61B <br> G01N |
| X | US 2015/126825 A1 (LEBOEUF STEVEN FRANCIS [US] ET AL) 7 May 2015 (2015-05-07) <br> * paragraphs [0005] - [0015], [0066] - [0068], [0080] - [0085] * <br> * figures 1-2 * | 1-15 | |

The present search report has been drawn up for all claims

| Place of search | Date of completion of the search | Examiner |
|---|---|---|
| The Hague | 29 January 2025 | Faymann, Juan |

EPO FORM 1503 03.82 (P04C01)

2

## ANNEX TO THE EUROPEAN SEARCH REPORT
## ON EUROPEAN PATENT APPLICATION NO.

EP 24 20 9691

This annex lists the patent family members relating to the patent documents cited in the above-mentioned European search report.
The members are as contained in the European Patent Office EDP file on
The European Patent Office is in no way liable for these particulars which are merely given for the purpose of information.

29-01-2025

| Patent document cited in search report | | Publication date | Patent family member(s) | | Publication date |
|---|---|---|---|---|---|
| US 2021174778 | A1 | 10-06-2021 | US | 2017112671 A1 | 27-04-2017 |
| | | | US | 2021174778 A1 | 10-06-2021 |
| US 2022084672 | A1 | 17-03-2022 | NONE | | |
| US 2017351221 | A1 | 07-12-2017 | US | 2017351221 A1 | 07-12-2017 |
| | | | WO | 2017207632 A1 | 07-12-2017 |
| CN 109770863 | A | 21-05-2019 | NONE | | |
| US 2015126825 | A1 | 07-05-2015 | EP | 2094152 A1 | 02-09-2009 |
| | | | EP | 2862504 A2 | 22-04-2015 |
| | | | EP | 3081155 A1 | 19-10-2016 |
| | | | HK | 1207811 A1 | 12-02-2016 |
| | | | US | 2008146890 A1 | 19-06-2008 |
| | | | US | 2014051948 A1 | 20-02-2014 |
| | | | US | 2014058220 A1 | 27-02-2014 |
| | | | US | 2014094663 A1 | 03-04-2014 |
| | | | US | 2014235967 A1 | 21-08-2014 |
| | | | US | 2014235968 A1 | 21-08-2014 |
| | | | US | 2014243617 A1 | 28-08-2014 |
| | | | US | 2014323829 A1 | 30-10-2014 |
| | | | US | 2015126825 A1 | 07-05-2015 |
| | | | US | 2015138556 A1 | 21-05-2015 |
| | | | US | 2015150469 A1 | 04-06-2015 |
| | | | US | 2015366471 A1 | 24-12-2015 |
| | | | US | 2018184916 A1 | 05-07-2018 |
| | | | US | 2018220902 A1 | 09-08-2018 |
| | | | US | 2018220903 A1 | 09-08-2018 |
| | | | US | 2018220904 A1 | 09-08-2018 |
| | | | US | 2018220905 A1 | 09-08-2018 |
| | | | US | 2018220906 A1 | 09-08-2018 |
| | | | US | 2018228381 A1 | 16-08-2018 |
| | | | US | 2019357777 A1 | 28-11-2019 |
| | | | US | 2020345243 A1 | 05-11-2020 |
| | | | US | 2021236003 A1 | 05-08-2021 |
| | | | WO | 2008088511 A1 | 24-07-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Global Burden of Disease Study. *Lancet*, 2023
- **ELLA ADOO-KISSI-DEBRAH**. *Air Pollution a factor in girl's death*, 16 December 2020
- Global Burden of Disease Study. 2021
- Lancet. Public Health, 2023, vol. 8, e839-49